# NOUVEAU FASCICULE DE BREVET EUROPEEN

(11) **EP 0 522 138 B2**
(45) Date de publication et mention de la décision concernant l'opposition: **14.02.2007**
(45) Mention de la délivrance du brevet: 19.03.2003
(21) Numéro de dépôt: 92904852.8
(22) Date de dépôt: 24.01.1992
(51) Int. Cl.: C07K 14/10, A61K 39/145, A61K 39/39

(54) **COMPOSITION VACCINALE CONTRE LA GRIPPE CONTENANT COMME ADDITIF DU CORE DE VIRUS GRIPPAL A EFFET SYNERGIQUE**
INFLUENZAIMPFSTOFF, DER ALS SINERGISTISCHER ZUSATZMITTEL INFLUENZAVIRUSINNENKÖRPER ENTHÄLT
INFLUENZA VIRUS VACCINE COMPOSITION CONTAINING INFLUENZA VIRUS CORE AS AN ADDITIVE HAVING A SYNERGISTIC EFFECT

(30) Priorité: 24.01.1991 FR 9100806
(43) Date de publication de la demande: 13.01.1993
(73) Titulaire: Sanofi Pasteur, 69367 Lyon Cedex 07 (FR)
(72) Inventeur: MOSTE-DESHAIRS, Catherine, F-69160 Tassin-la-Demi-Lune (FR); MEIGNIER, Bernard, F-69510 Thurins (FR)
(74) Mandataire: Le Coupanec, Pascale A.M.P.
(86) Numéro de dépôt international: PCT/FR1992/000066
(87) Numéro de publication internationale: WO 1992/013002

(56) Documents cités:
- EP-A- 0 041 880
- WO-A-86/04242
- WO-A-90/14361
- JOURNAL OF IMMUNOLOGY vol. 119, no. 6, Décembre 1977, pages 2073 - 2077; R.G.WEBSTER: 'POTENTIATION OF THE IMMUNE RESPONSE TO INFLUENZA VIRUS SUBUNIT VACCINES'

## Description

La présente invention a pour objet une composition vaccinale contre la grippe.

Le virus de la grippe est constitué d'une enveloppe lipoprotéique entourant un "core" nucléoprotéique. L'enveloppe contient notamment deux glycoprotéines, l'hémagglutinine (HA) et la neuraminidase (NA). Le core est un arrangement complexe de l'acide ribonucléique viral et de plusieurs protéines dites "internes" (polymérases, protéine de membrane (M) et nucléoprotéine (NP)).

On sait qu'actuellement la vaccination anti-grippale, même convenablement appliquée, ne protège pas complètement la totalité des sujets vaccinés ; voir par exemple Murphy et Webster, in "Virology", 2e édition (Fields et al. Eds) 1091-1152 (1990) en particulier p.1128.

Il est donc désirable d'améliorer les vaccins existants.

Les vaccins anti-grippaux actuellement utilisés sont des vaccins inactivés : ils peuvent être constitués de virions entiers, ou bien de virions soumis à un traitement avec des agents dissolvants des lipides (vaccins "splittés", encore appelés en langue française "vaccins fractionnés"), ou encore de glycoprotéines purifiées (vaccins "à sous-unités"). Ces vaccins inactivés protègent essentiellement en provoquant chez les receveurs la synthèse d'anticorps dirigés contre l'hénagglutinine. On sait que l'évolution antigénique du virus grippal par mutation résulte essentiellement de modifications de HA et de NA, tandis que les protéines internes sont peu modifiées. Il en résulte que les vaccins inactivés actuellement utilisés ne confèrent une protection efficace que vis-à-vis des souches dont les glycoprotéines de surface sont identiques ou antigéniquement très apparentées à celles des souches vaccinales. Pour obtenir un spectre antigénique suffisant, les vaccins sont obtenus à partir de plusieurs souches de virus ; ils contiennent généralement 2 souches de type A et une souche de type B. Pour adapter la composition des vaccins à l'évolution antigénique des virus grippaux, le choix des souches destinées à constituer les vaccins est revu annuellement en fonction des recommandations de l'Organisation Mondiale de la Santé ou de la Food and Drug Administration, ces recommandations étant basées sur les résultats d'une surveillance épidémiologique internationale. On sait que les souches virales recommandées peuvent être obtenues notamment auprès des organismes suivants :
- NIBSC (National Institute for Biological Standards and Control, London, UK)
- WIC (World Influenza Center, London UK)
- CDC (Center for Diseases Control, Atlanta, USA)
- CBER (Comity of Biological Evolution and Research, Washington, USA)

On a maintenant découvert qu'il est possible d'obtenir une composition vaccinale à effet synergique en associant à un vaccin anti-grippal classique à virions entiers ou un vaccin anti-grippal fractionné classique du core de virus grippal, ou une fraction active de core. Une fraction active core de virus grippal, ou une fraction active de core. Une fraction active de core est une fraction qui, utilisée comme additif à un vaccin anti-grippal classique, améliore l'effet de vaccination dudit vaccin. Ce qu'on entend ici par le terme "amélioration" sera précisé ci-après.

En outre, une protection contre des sous-types de virus non utilisés dans la préparation des composants du vaccin (vaccin classique + core ou fraction de core) peut être obtenue.

L'article de Webster *et al*., J. Immunol., Vol. 119, No6, 2073-2077 (1977) propose, pour remédier à la faible efficacité des vaccins à sous-unités lors d'une première vaccination, de les associer à des vaccins à virus entiers, cette association apportant une augmentation de la réponse anticorps. Ainsi, ce document décrit l'association de deux vaccins "classiques" tels qu'ils sont définis dans la présente demande.

La présente invention a donc pour objet une composition vaccinale contre la grippe contenant un vaccin anti-grippal classique à virions entiers ou un vaccin anti-grippal fractionné classique et contenant en outre, comme additif, des particules de core isolées d'au moins une souche de virus grippal et/ou une fraction isolée dudit core, cette composition étant telle que définie dans les revendications annexées.

Le vaccin classique formant le premier constituant de la composition vaccinale de l'invention peut être un vaccin à virions entiers ou un vaccin splitté. Il peut être obtenu au départ de virus cultivés en oeufs de poule embryonnés ou sur cellules.

Ces vaccins classiques peuvent être préparés selon des méthodes connues, qui sont rappelées par exemple par Murphy et Webster, ouvrage cité ci-dessus. D'autres précisions sont données ci-après.

Vaccin à virions entiers : il peut être préparé de la façon suivante : le-virus grippal, obtenu par culture en oeufs de poule embryonnés, ou par culture sur cellules, est concentré par ultrafiltration puis purifié par centrifugation zonale ou par chromatographie. Il est inactivé avant ou après purification par exemple par l'action du formol ou de la bêta-propiolactone.

Vaccin splitté : on peut le préparer comme suit : une suspension aqueuse du virus purifié obtenu comme ci-dessus, inactivé ou non, est traitée, sous agitation, par des solvants des lipides, tels que l'éther éthylique ou le chloroforme, associés à des détergents. La dissolution des lipides de l'enveloppe virale a pour effet de fragmenter les particules virales. On recueille la phase aqueuse qui contient le vaccin splitté, constitué essentiellement d'hémaglutinine et de neuraminidase, sans leur environnement lipidique d'origine, ainsi que du core ou des produits de dégradation de celui-ci. On procède ensuite à l'inactivation des particules infectieuses résiduelles, si celle-ci n'a pas déjà été effectuée. On peut opérer par exemple de façon analogue à celle décrite dans le brevet francais 2 201 079 (voir en particulier l'exemple 1).

Les vaccins classiques contiennent généralement de 10 à 15µg d'hémagglutinine de chacune des souches qui entrent dans leur composition.

Le vaccin anti-grippal classique formant le premier constituant de la composition vaccinale de l'invention peut provenir d'un virus de type A, B ou C, ou d'au moins deux de ces trois types. Il en va de même pour le core ou pour la fraction de core.

Le core, ou la fraction de core, peut être préparé à partir de virus de la même souche que le premier constituant de la composition, ou à partir de souche(s) différente(s), qui peuvent soit appartenir à un type différent (ou bien dans le cas du type A, à un sous-type différent), soit, à l'intérieur d'un même type ou sous-type, consister en isolat(s) différent (s) ou en réassortant(s) différent(s).

La nomenclature des virus de la grippe et leur classification en types et sous-types sont décrites par exemple dans WHO Bull. 58, 585-591 (1980) ainsi que Murphy et Webster, ouvrage cité ci-dessus.On sait en particulier que le type A inclut les sous-types H1N1, H2N2 et H3N2.

Dans la composition de l'invention, le premier et le second constituants, c'est à dire le vaccin classique et l'additif, sont réunis dans un même conteneur.

La composition de l'invention peut contenir les premier et second constituants, réunis en suspension dans un véhicule liquide approprié.

Les deux constituants de la composition vaccinale de l'invention, réunis peuvent aussi être présentés sous forme lyophilisée. On reconstitue alors la composition liquide par mélange avec un véhicule liquide usuel, au moment de l'emploi.

La composition de l'invention est présentée généralement sous la forme de doses vaccinales unitaires, constituées par une dose vaccinale du mélange des deux constituants.

Le second constituant de la composition de l'invention (core) peut être obtenu selon les méthodes connues, et en particulier par traitement du virus grippal à l'aide d'une protéase telle que la bromélaine. Un tel traitement permet de séparer les protéines d'enveloppe des particules de core ; voir par exemple Brand et Skehel, Nature, New. Biolog. Vol. 238, 145-147, 1972.

On peut employer d'autres enzymes ayant une action analogue à celle de la bromélaïne.

Le second constituant de la composition vaccinale de l'invention peut également être composé d'une fraction active de core de virus grippal, cette fraction étant une fraction protéique ou lipoprotéique, contenant au moins une protéine active de core (notamment la protéine M), ou encore un fragment actif de cette protéine. On désigne par l'expression "protéine active" ou "fragment actif' ou "fraction active" une protéine ou un fragment de cette protéine ou une fraction de core, qui est capable de participer à la protection induite par le vaccin, comme les particules de core elles-mêmes. Les fragments ou fractions actifs peuvent être déterminés par de simples expériences de routine, en retenant les fragments ou fractions qui, en association avec le premier constituant de la composition vaccinale, confèrent une protection supérieure à celle obtenue avec le premier constituant (vaccin classique) seul.

On notera que la protéine NP seule ne constitue pas une fraction active au sens donné dans la présente demande. L'association (M + NP) constitue une fraction active, ayant sensiblement l'activité de la protéine M qu'elle contient.

Les fractions de core, y compris une protéine de core ou les fragments de ladite protéine, en particulier une protéine M peuvent être préparés soit par culture de virus et extraction, soit par les méthodes du génie génétique, soit par synthèse peptidique, selon des méthodes connues en soi.

Le second constituant (additif) du vaccin de l'invention est en particulier une protéine M, ou protéine de membrane, encore appelée protéine de matrice. On sait qu'il existe deux protéines de matrice qui jouent un rôle dans l'assemblage du virus lors de sa réplication : la protéine M1, qui fait partie de la structure du virus, et la protéine M2, qui a été détectée dans le virus complet, mais dont une proportion importante n'est pas intégrée dans le virus mature. Dans la présente demande, l'expression "protéine M" désigne la protéine de matrice majoritaire dans le virus complet, c'est-à-dire la protéine M1, éventuellement en mélange avec d'autres protéines ou fractions de core.

Une fraction de core contenant la protéine M, et pouvant constituer l'additif au vaccin selon l'invention, peut être préparée selon les techniques connues de séparation et de purification des protéines ; on peut utiliser par exemple une méthode analogue à celle décrite par RUIGROK et al., Virology, 173, 311-316 (1989).

Ce procédé consiste principalement :
- à traiter une suspension de core à l'aide d'un tensioactif, par exemple un tensioactif non ionique, à une concentration suffisante et à un pH suffisamment acide pour favoriser la séparation des protéines M et NP dans l'étape suivante,
- à soumettre la solution ainsi traitée à une centrifugation à une vitesse suffisante pour que la protéine NP et d'éventuelles particules de core résiduelles s'accumulent dans le culot de centrifugation tandis que la protéine M reste dans le surnageant,
- à séparer le culot de centrifugation et recueillir le surnageant,
- et à concentrer, si désiré, le surnageant, pour obtenir une solution (fraction de core) constituant un additif pour une composition vaccinale selon l'invention.

Le tensioactif non ionique utilisé est par exemple un alkyl-phénol polyoxyéthyléné comme le Triton X 100 (Rohm & Haas), un alcool gras polyoxyéthyléné comme le Brij 36 T (Sigma) ou un alkyl-oside tel que l'octyl bêta-D glucopyranoside (ou octylglucoside, commercialisé par Sigma).

Le pH acide favorisant la séparation des protéines M et NP lors de l'étape de centrifugation est par exemple un pH de 4,8 environ. La centrifugation est effectuée par exemple à 85000g pendant 90 minutes.

L'étape de concentration finale du surnageant peut être réalisée notamment par ultrafiltration, en utilisant par exemple une membrane ayant un seuil de coupure de 10000 dalton. Le facteur de concentration, par exemple de l'ordre de 10 à 20, est évidemment choisi tel que l'additif soit présent en quantité efficace dans un volume compatible avec l'administration comme vaccin. On peut en outre éliminer si nécessaire le détergent par exemple par dialyse.

Le surnageant éventuellement concentré ainsi obtenu peut être utilisé comme additif, en quantité suffisante pour obtenir une amélioration de la vaccination. On peut évaluer la quantité de cet additif par exemple en la rapportant à la quantité de protéine M qu'il contient.

La détection et le dosage de protéine M peuvent être effectués par exemple à l'aide d'anticorps spécifiques, selon les techniques immunologiques classiques, par exemple un test ELISA, comme cela sera précisé ci-après.

Les fractions de core peuvent être également des particules de core délipidées pouvant être obtenues par traitement ménagé du virus par au moins un tensioactif, généralement utilisé à faible concentration, par exemple des tensioactifs non ioniques (tels que ceux commercialisés sous la dénomination NONIDET P40 ou TRITON X100), ou certains tensioactifs cationiques (tels que le bromure d'hexadécyl triméthyl ammonium). Les concentrations convenables peuvent être déterminées dans chaque cas par des expériences de routine : il s'agit des concentrations qui laissent subsister le core sous forme de particules ; voir par exemple Bachmayer, Virology 69, 511-522, 1976; et Rigg et al., J. Gen. Virol. 70, 2097-2109 (1989).

Les cores délipidés peuvent être alors séparés et/ou purifiés selon les méthodes usuelles, notamment par centrifugation.

Lorsque l'additif au vaccin selon l'invention est sous la forme de particules de core, il peut donc s'agir de particules de core obtenues par l'action de la bromelaïne (ou analogues) et/ou de particules de core délipidées. Dans les deux cas, il s'agit de particules qui sont sensiblement exemptes d'hémagglutinine et de neuraminidase.

La composition vaccinale de l'invention peut être administrée chez l'homme ou chez les animaux susceptibles de souffrir de la grippe, notamment les espèces équines, porcines et aviaires. Les doses de composition à administrer sont les doses usuelles pour ce genre de vaccin, et peuvent éventuellement, chez l'animal, être déterminées dans chaque cas par des expériences de routine.

Par exemple, les doses unitaires, pour le premier constituant (vaccin classique) sont généralement définies par leur contenu en hémagglutinine. Elles correspondent pour chacun des 3 types de vaccins (vaccin à virion entier, vaccin à sous-unités et vaccin splitté) généralement à 1-20 µg, et en particulier 5-20 µg, par exemple 10-15 µg d'hémagglutinine de chacune des souches vaccinales qui les composent.

Ces quantités d'hémagglutinine peuvent être mesurées selon la méthode d'immunodiffusion radiale décrite par Wood et coll, Journal of Biological Standardization, 5, 237-247 (1977).

La quantité d'additif, dans la composition vaccinale de l'invention, est une quantité efficace prédéterminée, qui est suffisante pour provoquer, chez l'espèce animale concernée, une amélioration statistiquement significative de l'efficacité de la vaccination.

La quantité d'additif à utiliser avec une dose unitaire de vaccin est par exemple une quantité suffisante pour apporter statistiquement une amélioration d'au moins 5 %, et en particulier d'au moins 10 %, de l'efficacité de la vaccination, évaluée sur au moins un critère reconnu d'efficacité de la vaccination. L'efficacité de la vaccination peut être déterminée par example par des études épidémiologiques de populations vaccinées avec un vaccin classique anti-grippal classique à virions entiers ou un vaccin anti-grippal fractionné classique et de populations vaccinées avec le vaccin classique anti-grippal classique à virions entiers ou un vaccin anti-grippal fractionné classique et l'additif, et éventuellement de populations non vaccinées. Les critères retenus pour l'appréciation de l'efficacité de la vaccination sont ceux qui sont couramment utilisés par les spécialistes dans ce type d'études et notamment :
- la proportion d'individus vaccinés atteints d'une affection grippale, par rapport au nombre total d'individus vaccinés, dans une région où une épidémie de grippe s'est effectivement développée ;
- ou la gravité ou la durée de la maladie grippale,
- ou par le nombre, la gravité ou la durée des complications,
- ou la protection contre un sous-type autre que le ou les sous-types des constituants du vaccin (vaccin classique + additif).
- ou encore l'amélioration de la qualité du vaccin peut être évaluée à travers une augmentation significative de la réponse immunitaire, appréciée par exemple par le pourcentage de sujets séro-convertis, par le taux des anticorps dirigés contre le virus de la grippe ou ses composants, ou encore par des tests mesurant la réponse immunitaire de type cellulaire au virus de la grippe ou à ses composants.

Avec certaines espèces, notamment d'animaux de laboratoire, ou avec des volontaires, il est également possible de déterminer l'efficacité d'une vaccination vis-à-vis d'infections expérimentales.

Les doses unitaires pour le second constituant (additif) peuvent contenir généralement, et notamment chez l'homme, de 1 à 100 µg, (en particulier 2 à 100 µg, ou 5 à 100 µg) et notamment de 2 à 50 µg, par exemple de 5 à 30µg de particules de core, ou une quantité équivalente de la fraction de core utilisée, (c'est-à-dire une quantité correspondant à la quantité de ladite fraction contenue dans ladite quantité de particules de core, ou encore une quantité de ladite fraction ayant la même activité, dans la composition vaccinale, que ladite quantité de particules de core, cette activité étant bien entendu définie en relation avec l'efficacité du vaccin selon au moins l'un des critères indiqués ci-dessus).

Les quantités de core indiquées sont exprimées en quantités de protéines totales mesurées par exemple selon la méthode de Bradford mentionnée dans la partie expérimentale ci-après.

La mesure des quantités de core peut être effectuée par séparation des particules en fonction de leur densité et comparaison avec une gamme étalon de core.

On peut procéder de façon analogue pour les particules de core délipidées qui, contrairement aux particules de core obtenues par traitement à la bromélaïne, ont une densité supérieure à celle du virion.

Lorsque l'additif est au moins une protéine M, ou une fraction de core contenant de la protéine M, la dose unitaire de composition vaccinale contient de préférence au moins 3-5 µg, et en particulier au moins 7-10 µg de protéine M ajoutée (c'est-à-dire en sus de la protéine M libre éventuellement déjà présente dans le vaccin classique, notamment lorsqu'il s'agit d'un vaccin splitté). Les quantités de protéine M ajoutée sont évaluées ici notamment à l'aide d'un essai immunologique selon la technique ELISA. Les quantités de protéine M dosée par ELISA sont déterminées par comparaison avec un étalon de protéine M purifiée (dosée par exemple à l'aide de la méthode à l'acide bicinchonique). On peut aussi procéder par comparaison avec la teneur en protéine M d'un virus grippal purifié soumis à l'action d'un détergent, en admettant que la protéine M représente 50 % en poids des protéines totales du virus. Les essais ELISA sont effectués, sur les préparations testées ou sur les préparations témoins de protéine M ou de virus, en solution contenant par exemple 0,1 % de dodécyl sulfate de sodium (SDS). Les protéines virales totales sont dosées par toute méthode appropriée, par exemple la méthode de Bradford mentionnée dans la partie expérimentale.

On sait que les vaccins à virions entiers, ainsi que les vaccins à sous-unités, sont pratiquement exempts de protéine M libre (c'est-à-dire en-dehors des particules de virus ou de core). Les vaccins splittés classiques contiennent certaines quantités de protéine M, ces quantités étant variables et dépendant principalement de la technique de préparation utilisée.

Ainsi, avec la connaissance de la technique de préparation utilisée, et donc des quantités de protéine M libre en solution normalement présentes dans la composition de vaccin obtenue, il est facile de déterminer les quantités de protéine M éventuellement ajoutées dans une composition de vaccin donnée.

En outre, on a constaté expérimentalement que la protéine M ajoutée a généralement une densité (mesurée par exemple en gradient de saccharose), différente de celle de la protéine M déjà présente dans la préparation de vaccin splitté.

La composition de l'invention peut être administrée par voies sous-cutanée ou intra-musculaire, ou encore par voie nasale ou orale, ou bien sous forme d'aérosol.

Son administration peut être associée à celle d'autres vaccins et/ou d'adjuvants.

La composition peut être en outre utilisée lors d'administrations de rappel, par exemple de 1 à 3 mois après une première vaccination.

L'invention a également pour objet l'utilisation, dans la préparation d'une composition vaccinale contre la grippe contenant un premier constituant correspondant à un vaccin anti-grippal classique à virions entier ou un vaccin anti-grippal fractionné classique d'un second constituant (additif) comprenant des particules de core, ou une fraction purifiée de core d'au moins u virus grippal, lesdits premier et second constituants étant tels que définis ci-dessus et étant présents dans un même conteneur pour obtenir une composition vaccinale à effet synergique.

Les exemples suivants illustrent l'invention sans toutefois la limiter.

### EXEMPLE 1 : Obtention de core viral purifié

On a utilisé la souche réassortante de virus grippal NIB16 (A/H1N1) issue du croisement entre la souche sauvage A/Taïwan/1/86 (A/H1N1) et le réassortant X31 (A/H3N2) lui-même obtenu en croisant la souche A/Aïchi/2/68 (A/H3N2) avec le virus A/Porto-Rico/8/34 (A/H1N1).

La souche NIB16 peut être obtenue auprès du NIBSC.

Les suspensions virales ont été préparées par multiplication sur oeufs de poule embryonnés, concentration par ultrafiltration et purification sur gradient de saccharose comme décrit dans la demande de brevet français n° 2 201 079.

Pour extraire le core, on soumet le virus purifié, mis en suspension en tampon phosphate pH 7,4 (tampon PBS) à deux ou trois traitements successifs par la bromélaïne (Sigma) à 37°C en tampon tris 0,1 M pH 7,5, EDTA 1 mM, bêta-mercaptoéthanol 50 mM. Pour le premier traitement avec la protéase, on utilise la suspension virale ajustée pour contenir 2 mg de protéines par ml de tampon et on ajoute 1 mg/ml de bromélaïne. Après 2 heures d'incubation à 37°C et dilution avec une solution aqueuse de NaCl 0,1 M, on soumet la préparation à une séparation par ultra-centrifugation à 120 000 g, pendant 90 min, à + 4°C. Pour les deuxième et (éventuellement) troisième traitements, l'incubation est faite en utilisant la bromélaïne à 2 mg/ml (concentration finale) pendant 16 heures. Les traitements de centrifugation sont les mêmes que lors du premier traitement et les culots sont repris en tampon PBS.

On soumet la solution de core obtenue à une purification par ultra-centrifugation isopycnique sur un gradient linéaire de saccharose 20-60% (p/p) en tampon PBS, à 100 000 g pendant 16 h, à + 4°C. Les fractions contenant le core viral sont diluées au 1/3 par du tampon PBS, puis soumises à une ultra-centrifugation à 120 000 g pendant 90 min, à + 4°C. Le culot de centrifugation est repris par du tampon PBS pH 7,4 additionné d'azoture de sodium à 0,01 %. La solution peut être conservée par congélation à moins 20°C.

La préparation de core purifié ainsi obtenue présente les caractéristiques suivantes :
- la proportion d'hémagglutinine, par rapport aux protéines totales, est au maximum de 4 %, cette proportion étant évaluée par électrophorèse en gel de polyacrylamide (Laemmli, Nature, 227, 680-685, 1970) ou par technique ELISA ;
- activité hémagglutinante : inférieure à 0,01% de celle du virus de départ (évaluation par hémagglutination selon la méthode décrite par Palmer et al., Advanced laboratory technicals for immunological diagnostic U.S. Dept H1th Ed. Welfare, P.H.S. Atlanta, Immunology ser. n° 6, Procedural guide Part 2, hemagglutination inhibition test, 1975, 25-62).
Le vaccin final est préparé par dilution en tampon PBS, comme indiqué à l'exemple 2 ci-après.

### EXEMPLE 2 : Préparation du vaccin et étude pharmacologique

On utilise comme premier constituant de la composition vaccinale un vaccin monovalent splitté et inactivé, obtenu avec la souche NIB16.

L'hémagglutinine de NIB16 est analogue à celle de la souche A/Singapore/6/86 (A/H1N1).

Ce vaccin splitté a été obtenu par traitement du virus avec un mélange de Polysorbate 80 et d'éther selon la méthode décrite dans le brevet français 2 201 079 (exemple 1).

Le deuxième constituant (core) a été obtenu selon le procédé décrit à l'exemple 1 ci-dessus.

Le vaccin monovalent et le core ont été dilués et mélangés en tampon PBS pour apporter sous un volume de 0,5 ml les combinaisons et les doses indiquées dans les tableaux 1 et 2 ci-après. La composition ainsi obtenue a été injectée à des souris OF1 (IFFA-CREDO France) âgées de six semaines, par voie sous-cutanée.

Les doses de vaccin splitté et de core utilisées sont exprimées en µg de protéines totales déterminées par un dosage protéïque colorimétrique, par comparaison à une gamme étalon de sérum albumine bovine, selon la méthode décrite par Bradford (Anal. Biochem, 1976, 72, 248-354), à l'aide du Kit Bio-rad.

Un mois après la vaccination, on a infecté les souris avec la souche de virus grippal A/Wilson Smith/33 (A/H1N1), obtenue auprès du Centre Mondial de la Grippe de Londres. Cette souche a été choisie pour les épreuves virulentes car elle est létale pour les souris non immunisées. Elle a été administrée par voie nasale, à raison de 20 doses létales 50% (DL50) dans 30µl par souris sous anesthésie. Les souris ont ensuite été observées quotidiennement pendant 3 semaines.

Les résultats concernant les survies enregistrées dans les différents groupes expérimentaux ont été rassemblés dans les tableaux 1 et 2. Dans les expériences du tableau 1, aucune souris témoin (non vaccinée) n'a survécu. Le core viral administré seul n'a, au mieux, qu'un effet protecteur limité (10 à 20%) et le vaccin splitté injecté seul ne protège que 30 à 50% des souris. On voit que plusieurs des combinaisons de vaccin splitté et de core ont donné lieu à une protection synergique à partir des concentrations de 3µg de vaccin splitté associés à 90µg de core, ou encore 10µg de vaccin splitté associés à 10µg de core.

L'augmentation de la survie obtenue en associant vaccin splitté et core est statistiquement significative. Les résultats ont été soumis à une analyse de variance (test F de FISCHER-SNEDECOR) qui a mis en évidence que l'addition de core a un effet synergique statistiquement significatif (p = 0,027) sur la survie des souris vaccinées.

L'expérience a été répétée en réduisant la gamme des quantités de core essayées en association avec le vaccin splitté. Les résultats sont présentés dans le tableau 2. On voit, d'après les résultats du tableau 2, que l'adjonction au vaccin de 3µg de cote ou davantage augmente systématiquement le pourcentage des souris qui survivent à l'épreuve (synergie de protection hautement significative : p test F = 0,009)

**TABLEAU 1**

| Souris survivantes / Souris éprouvées après immunisation avec, par souris : | | | | |
|---|---|---|---|---|
| Vaccin | additionné de core (µg) : | | | |
| splitté (µg) | 0 | 10 | 30 | 90 |
| 0 (PBS seul) | 0/10 | 1/10 | 2/10 | 2/10 |
| 3 | 3/10 | 2/10 | 3/10 | 8/10 |
| 10 | 4/10 | 7/9 | 10/10 | 7/10 |
| 30 | 5/10 | 10/10 | 7/10 | 8/10 |

**TABLEAU 2**

| Souris survivantes / Souris éprouvées après immunisation avec, par souris : | | | |
|---|---|---|---|
| Vaccin | additionné de core (µg) : | | |
| splitté (µg) | 0 | 3 | 10 |
| 0 (PBS) | 1/10 | 0/10 | 1/10 |
| 3 | 2/10 | 8/10 | 10/10 |
| 10 | 6/10 | 10/10 | 10/10 |

### EXEMPLE 3 : Détection et quantification du core de virus grippal.

Les vaccins grippaux de l'invention sont susceptibles de contenir, en proportions variable selon leur mode de préparation, du core grippal (délipidé ou non) et des virions entiers ou des sous-unités protéiques.

La méthode choisie pour doser le core utilise la différence de densité de ces éléments, mise en évidence par centrifugation isopycnique en gradient linéaire de saccharose (Brand et Skehel, article cité ci-dessus).

Pour cela les échantillons à analyser sont déposés dans des tubes à ultracentrifugation à la surface d'un gradient de saccharose préformé. Dans le cas présent, ou a utilisé des tubes de 14 ml avec 12 ml de gradient 20-60 % (p/p en PBS). La dose de vaccin déposée a un volume de 1 ml.

Les échantillons sont ensuite soumis à ultracentrifugation pendant 16 heures à 100 000 g (à + 4°C), puis le contenu du tube est fractionné de la surface vers le fond en 14 aliquotes. Au cours du fractionnement, la densité optique est mesurée en continu à 254 mm.

On obtient un diagramme où chaque pic correspond à une-population de particules de densité déterminée. L'appareillage permet de repérer la correspondance entre la position d'un pic sur le graphique et la fraction où il se trouve. La mesure au réfractomètre d'Abbe permet de connaître le pourcentage de saccharose de chaque fraction, à partir duquel des tables de conversion (Handbook of chemistry and physics, 68th edition, Ed.R.C. Weast, CRC Press Inc.) donnent la densité apparente des particules. De manière caractéristique, la densité du core viral (obtenu selon le procédé de l'exemple 1) est de 1,15-1,16 g/cm³ et celle du virus de 1,19-1,20 g/cm³ ; cela correspond à des concentrations de saccharose de 35-37 % et 42-44 % respectivement.

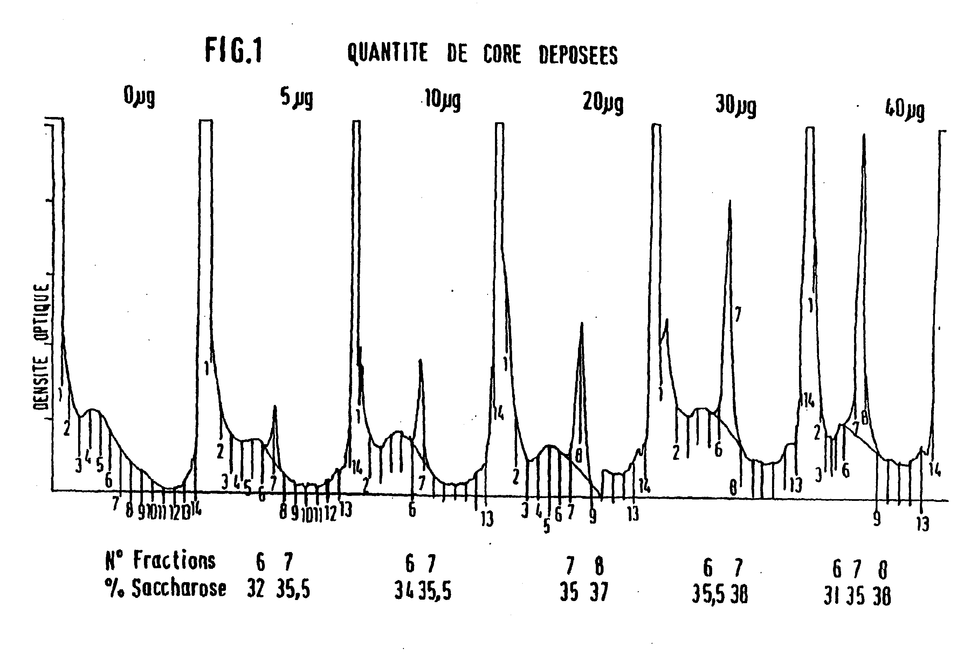

Les résultats de l'analyse de densité d'une gamme étalon de core sont représentés sur la figure 1.

Dans les graphiques de la figure 1 on a indiqué en abscisses les numéros des fractions (et les concentrations de saccharose correspondantes) et on a représenté en ordonnées la densité optique (DO) en unité arbitraire. Les conditions d'enregistrement (matériel ISCO) sont les suivantes : longueur d'onde de détection 254 nm, sensibilité 0,2 de DO (pleine échelle), débit de la collecte : 3 ml/cm.

Les graphiques présentés dans la figure 1 ont été obtenus en testant des quantités croissantes de core en solution dans le tampon PBS. On constate que la surface délimitée par les pics est proportionnelle à la quantité de core. On peut établir une corrélation qui permet ensuite d'employer la méthode pour les dosages.

Pour confirmer que le pic observé pour une concentration de saccharose de 35-37 % est du core viral, on peut employer, (après un traitement dénaturant de l'échantillon par le SDS à 2 %, à 90°C, pendant 3 min), l'électrophorèse en gel de polyacrylamide (Laemmli, article déjà cité), qui montre, après coloration à l'argent, la présence des protéines de core NP et M.

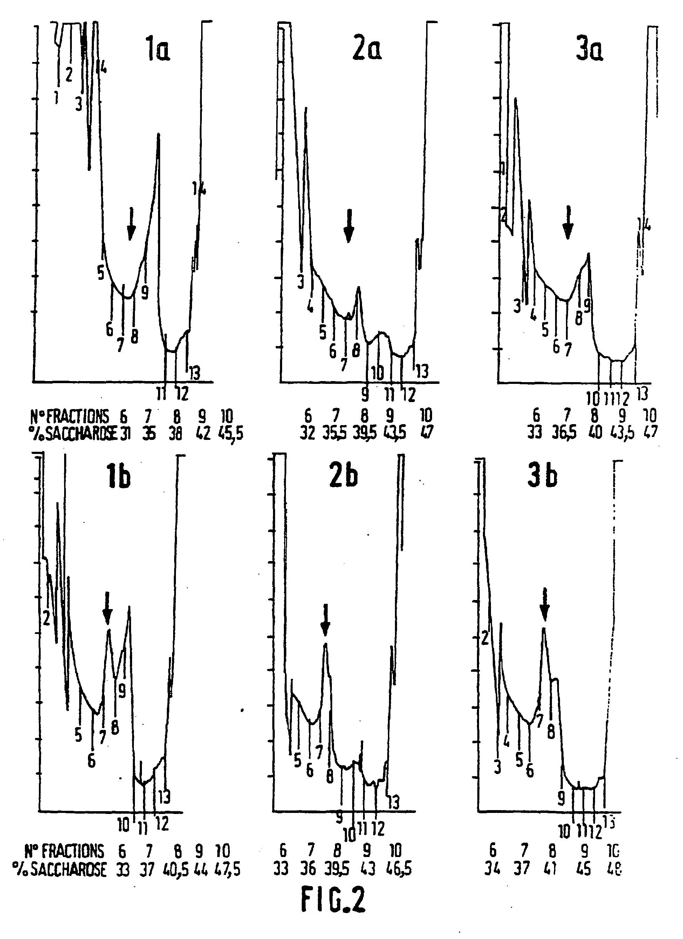

On a représenté dans la figure 2 les diagrammes obtenus avec 3 vaccins différents : un vaccin à virions entiers, commercialisé sous la marque Vaccin Grippal Ronchèse (VGR), et deux vaccins splittés avec des techniques différentes commercialisés sous les marques VAXIGRIP et MUTAGRIP, ces diagrammes étant établis selon les mêmes principes, et dans les mêmes conditions d'enregistrement, que ceux exposés à propos de la figure 1. La figure 2 permet une comparaison des profils de vaccins grippaux (une dose) avant (a) et après (b) addition de 10 µg de core viral. Sur la figure 2, les graphiques 1 correspondent au vaccin à virions entiers, et les graphiques 2 et 3 aux vaccins splittés (Vaxigrip et Mutagrip respectivement). Les profils varient d'un vaccin à l'autre mais tous sont dépourvus de core.

L'addition de core (10 µg/dose) (graphiques 1 b, 2b 3b) est clairement identifiable par l'apparition d'un nouveau pic dans les fractions contenant le saccharose à 36-37 %.

### Exemple 4

### Préparation d'une fraction de core contenant une protéine de matrice (protéine M); essais de vaccination et de dosage.

** Les références bibliographiques figurant dans cet exemple sont rassemblées à la fin de l'exemple.

Cette fraction est extraite à partir de core viral purifié selon une technique adaptée de RUIGROK et coll. (1989). Le core est mis en suspension dans du tampon PBS ajusté à pH 4,8 à l'aide d'une solution d'acide citrique à 0,25 M. A cette étape, on peut ajouter un inhibiteur de protéases tel que le TLCK (Sigma, solution à 1 mg/ml en tampon acétate 50 mM pH5), afin d'éviter une dégradation ultérieure de la protéine M. Le core est ensuite soumis à un traitement détergent par une solution mère de Lubrol (Brij 36 T, Sigma) à 10 %, les concentrations finales de core, de Lubrol et éventuellement de TLCK étant respectivement ramenées à 0,1 mg/ml, 0,5 % et 50 µg/ml, par addition de tampon PBS ajusté à pH 4,8 à l'aide d'acide chlorhydrique 1,24 N. Le mélange est homogénéisé par agitation douce à la température ambiante pendant 1 min, puis soumis à ultra-centrifugation à 85 000 g pendant 90 min, à + 4°C. Le culot de centrifugation contenant la nucléoprotéine (NP) est remis en suspension en tampon PBS pH 7,4, tandis que le surnageant, qui contient la protéine M, est concentré 10 à 20 fois par ultrafiltration (cellule Amicon, membrane avec seuil de coupure de 10 000 daltons). Les protéines sont stockées à - 20 °C. Elles sont dosées à l'acide bicinchonique (Smith, Krohn et al., 1985) au moyen du Kit Micro BCA de Pierce. Leur pureté est contrôlée en routine par électrophorèse en gel de polyacrylamide à 12,5 % suivie d'une coloration au bleu de Coomassie (Phast system, Pharmacia). Aucune contamination n'est visible en ce qui concerne la protéine de matrice, ce qui indique un degré de pureté de celle-ci d'au minimum 90%.

### Test de protection sur souris

- Des groupes de souris BALB/c (provenance : IFFA-Credo, France), âgées de 6 semaines ont été immunisées avec des préparations de Vaxigrip (monovalent A/H1N1 NIB16) de protéine M, ou par des associations de Vaxigrip et de protéine M (voir doses utilisées dans les tableaux de résultats). Les différentes préparations ont été administrées par voie sous-cutanée sous 0,5 ml, sans adjuvant et en une seule injection. Les souris ont été éprouvées 4 à 5 semaines après immunisation avec 5 doses létales 50 % (DL50) de la souche A/H1N1 A/WS/33 inoculée sous 30 µl par voie intra-nasale, sous anesthésie profonde des animaux par un mélange de kétamine-Xylazine. Les résultats sont présentés comme le tableau de survie des souris, trois semaines après l'épreuve virulente.
Les deux tableaux présentés correspondent à deux séries d'expériences réalisées avec la même préparation de protéine M du virus NIB16. Ils montrent que l'association Vaxigrip (monovalent NIB16) -- préparation de protéine de matrice confère une protection améliorée.
Rappelons que les doses de vaccin, de core et de protéines sont exprimées ici en µg de protéines totales déterminées, pour le vaccin et le core par la technique de Bradford (1976), et pour la protéine M par le dosage à l'acide bicinchonique (Smith, Krohn et al., 1985, référence citée ci-dessus).

**TABLEAU 3**

| Nb de souris survivantes / Nb de souris éprouvées (10 sauf indications contraires) | | | |
|---|---|---|---|
| Protéine M de NIB 16 (µg) | | | |
| Vaccin NIB16 (µg) | 0 | 5 | 15 |
| 0 | 0 | 1/9 | 1 |
| 10 | 0 | 7 | 7/9 |

**TABLEAU 4**

| Nb de souris survivantes / 10 souris éprouvées | | |
|---|---|---|
| Vaccin NIB16 | | Protéine NIB16 |
| (µg) | 0 | M(5µg) |
| 0 | 1 | 0 |
| 10 | 2 | 9 |

- Les souris peuvent aussi être immunisées en utilisant des préparations de vaccin à virions entiers. Le tableau ci-dessous présente la survie des souris BALB/c immunisées à l'âge de six semaines avec des préparations de vaccin trivalent VGR à virions entiers (Vaccin Grippal Ronchèse de la saison 1990-1991). La dose de vaccin trivalent utilisée est d'environ 5 µg d'hémagglutinine du virus NIB16 par souris : elle a été quantifiée par immuno-diffusion radiale selon la technique de Wood et coll. déjà mentionnée ci-dessus, et correspond à la quantité présente dans 10 µg de protéines totales de monovalent Vaxigrip NIB16 précédemment utilisé. Les résultats montrent que l'effet d'amélioration de la protection par addition de la préparation de protéine M s'observe également avec un vaccin à virus entier :

**TABLEAU 5**

| Nb de souris survivantes / 10 souris éprouvées | | | |
|---|---|---|---|
| Vaccin VGR à virions entiers | Protéine M NIB16 (µg) | | |
| (µg HA NIB16) | 0 | 5 | 15 |
| 0 | 0 | 0 | 0 |
| 5 | 2 | 4 | 7 |

### Dosage de la protéine M par ELISA

### - Principe :

La technique utilisée est un test ELISA sandwich non-compétitif, adapté de Bucher, Kharitonenkov et al., (1987), Donofrio, Coonrod et al., (1986) et Hjerten, Sparrman et al., (1988). Elle consiste à capter la protéine M des échantillons à doser (par exemple virus grippal, vaccin, core, protéines purifiées) à l'aide d'immunoglobulines spécifiques de M préalablement adsorbées sur des plaques de microtitration ; la présence de la protéine est ensuite évaluée grâce à une succession d'étapes qui conduisent à une réaction colorimétrique proportionnelle à la quantité d'antigène présente.

### - Réactifs immunologiques utilisés :

Immunoglobulines totales anti-protéine M du virus grippal : ces immunoglobulines spécifiques ont été obtenues à partir de sérum de lapins hyperimmunisés par 3 injections respectivement de 100, 75 et 75 µg de protéine M préparée comme décrit précédemment ; ces injections ont été réalisées à un mois d'intervalle par voie intra-musculaire en présence d'adjuvant de Freund (adjuvant complet pour la première injection et incomplet pour les injections ultérieures). Les immunoglobulines totales ont ensuite été précipitées au sulfate d'ammonium à 35 % de saturation.
. Anticorps monoclonal murin du commerce (Serotec) spécifique de la protéine M
. Immunoglobulines de chèvre anti-immunoglobulines de souris, couplées à la peroxydase (Jackson ImmunoResearch).

### - Solutions :

- Tampon de dilution constitué de PBS pH7,2 additionné de 0,05 % de tween 20 et de 5% de lait écrémé en poudre (Régilait).
- Solution de lavage des plaques constituée de tampon PBS additionné de 0,05 % de tween 20.

### - Méthode :

Elle est réalisée en plaques de microtitration (Nunc).

Chaque réactif est ajouté sous un volume de 100 µl par cupule ; à partir de l'étape de saturation et jusqu'à celle de la révélation, les plaques subissent systématiquement 4 rinçages successifs à l'aide de la solution de lavage.

Les immunoglobulines totales de lapin anti-protéine M sont déposées à la concentration de 1 µg/ml en tampon carbonate de sodium 50 mM pH 9,6, et sont incubées pendant une nuit à + 4°C.

Une étape de saturation des cupules est ensuite effectuée pendant 1 h à 37 °C à l'aide du tampon de dilution.

Les échantillons à doser sont alors déposés sous la forme de dilutions de raison 2 effectuées dans le tampon de dilution additionné de 0,1 % de SDS.

Après un contact de 2 h à 37°C, l'anticorps monoclonal de souris spécifique de la protéine M, dilué à 1/1000 dans le tampon de dilution additionné de 0,1 % de SDS, est ajouté et on laisse incuber pendant 1 h à 37 °C.

La fixation de l'anticorps monoclonal anti-M sur la protéine est mise en évidence par addition des anticorps anti-immunoglobulines de souris marqués à la peroxydase dilués à 1/1000 dans le tampon de dilution.

Après 1 h à 37°C et après une dernière série de lavages des plaques, la réaction est révélée par addition de la solution tampon citrate 20 mM, pH 5,6 contenant du perborate de sodium, substrat de la peroxydase (Sigma), additionné d'oPD (dichlorhydrate d'orthophénylènediamine, Sigma) utilisé à la concentration de 0,4 mg/ml. La réaction est stoppée après une incubation de 20 min à température ambiante par addition de 50 µl d'acide sulfurique 4N. La lecture de la réaction est réalisée au moyen d'un lecteur de plaques ELISA (MR 5000 Dynatech) qui mesure l'absorbance du milieu réactionnel dans les cupules à la longueur d'onde de 490 nm.

### ** Références bibliographiques :

- Bachmayer, H (1975). "Selective solubilization of hemagglutinin and neuraminidase from influenza viruses" Intervirology 5, 260-272.
- Bradford, M.M. (1976). "A rapid and sensitive method for the quantitation of microgram quantities of proteins utilizing the principle of protein-dye binding". Anal Biochem 72, 248-254.
- Bucher, D.J. Kharitonenkov, I.G., Wajeed-Khan, M., Palo, A., Holloway D. and Mikhail A. (1987) "Detection of influenza viruses through selective adsorption and detection of the M protein antigen". J. of Immunol. Methods 96, 77-85.
- Donofrio, J.C., Coonrod, J.D., Karathanasis, V. and Coelingh, K.V.W. (1986). "Electroelution for purification of influenza A matrix protein for use in immunoassay". J. of Immunol. Methods 13, 107-120.
- Hjerten, S., Sparrman, M. and Liao, J. (1988). "Purification of membrane proteins in SDS and subsequent renaturation". Biochim, Biophys, Acta, 939, 476-484.
- Jennings, R., Smith, T.L., Spencer, R.C., Mellersh, A. M., Edey, D., Fenton, P., et al (1984). "Inactivated influenza virus vaccines in man : a comparative study of subunit and split vaccines using two methods for assessment of antibody responses." Vaccine, 2, 75-80.
- Ruigrok, R.W.H., Calder, L. J. and Wharton, S.T.A. (1989). "Electron Microscopy of the influenza Virus Submembranal Structure". Virology, 173, 311-316.
- Smith, P.K., Krohn, R.I., Hermanson, G.T., Mallia, A.K., Gartner, F.H., Provenzano, M.D., et al (1985). "Measurement of protein using bicinchonic acid'. Anal, Biochem., 150, 76-85.

### Exemple 5

### Amélioration de la protection contre un sous-type de virus grippal par utilisation, d'un vaccin de sous-type différent additionné de core.

Des groupes de souris OF1 mâles âgées de 6 semaines ont été immunisées avec des préparations de Vaxigrip monovalent A/H3N2 X 97, de core du virus A/H3N2 X 97, ou par des associations de Vaxigrip et de core. Les différentes préparations ont été administrées par voie sous-cutanée sous un volume de 0,5 ml, sans adjuvant et en une seule injection. Les souris ont été éprouvées 5 semaines après immunisation avec une dose correspondant à 20 doses létales 50 % (DL50) de la souche A/H1N1 A/WS/33 inoculée sous un volume de 30 µl par voie intra-nasale, sous anesthésie profonde des animaux par un mélange de kétamine-Xylazine.

Les résultats sont résumés dans le tableau suivant :

**TABLEAU 6**

| Nb de souris survivantes / Nb souris éprouvées (10 sauf indications contraires) | | | | |
|---|---|---|---|---|
| | Core X97 ajouté (µg) | | | |
| vaccin X97 (µg) | 0 | 3 | 10 | 30 |
| 0 | 0/9 | 0/9 | 0 | 0 |
| 8 | 0 | 0 | 0 | 0/9 |
| 10 | 1 | 3 | 3 | 1 |
| 30 | 0 | 1 | 4 | 7 |

De façon attendue, le vaccin A/H3N2 X97 ne confère pas de protection vis-à-vis d'une épreuve à virus A/H1N1. Un effet surprenant de synergie de protection est par contre observé lorsque le vaccin est associé à du core.

## Revendications

1. Composition vaccinale contre la grippe contenant un mélange d'un premier et d'un second constituants, ledit premier constituant étant formé d'un vaccin anti-grippal classique à virions entiers, et ledit second constituant étant un additif à effet synergique choisi parmi des particules de core isolées provenant d'au moins une souche de virus grippal, une fraction isolée de core contenant de la protéine M d'au moins une souche de virus grippal, une protéine M d'au moins une souche de virus grippal, et un fragment à effet synergique de protéine M d'au moins une souche de virus grippal.

2. Composition vaccinale contre la grippe contenant un mélange d'un premier et d'un second constituants, ledit premier constituant étant formé d'un vaccin anti-grippal fractionné classique, et ledit second constituant étant un additif à effet synergique choisi parmi des particules de core isolées provenant d'au moins une souche de virus grippal, une fraction isolée de core contenant de la protéine M d'au moins une souche de virus grippal, une protéine M d'au moins une souche de virus grippal, et un fragment à effet synergique de protéine M d'au moins une souche de virus grippal.

3. Composition selon l'une quelconque des revendications précédentes, **caractérisée par le fait que** ledit additif comprend du core obtenu après élimination des protéines d'enveloppe par traitement d'un virus grippal à l'aide d'une protéase.

4. Composition selon la revendication 3, **caractérisée par le fait que** ladite protéase est la bromélaïne.

5. Composition selon l'une quelconque des revendications 1 à 4, **caractérisée par le fait que** ledit additif comprend une fraction isolée de core de virus grippal contenant de la protéine M, ou comprend un fragment à effet synergique de la protéine M.

6. Composition selon la revendication 5, **caractérisée par le fait que** ledit additif comprend des particules de core délipidées.

7. Composition selon l'une quelconque 1 à 5, **caractérisée par le fait que** ledit additif comprend du core obtenu par traitement ménagé d'un virus grippal à l'aide d'au moins un tensioactif, puis séparation dudit core selon les méthodes usuelles.

8. Composition selon la revendication 5, **caractérisée par le fait que** ladite fraction de core peut être obtenue par un procédé consistant à :
- traiter une suspension de core à l'aide d'un tensioactif, par exemple un tensioactif non ionique, à une concentration suffisante et à un pH suffisamment acide pour favoriser la séparation des protéines M et NP dans l'étape suivante,
- soumettre la solution ainsi traitée à une centrifugation à une vitesse suffisante pour que la protéine NP et d'éventuelles particules de core résiduelles s'accumulent dans le culot de centrifugation tandis que la protéine M reste dans le surnageant,
- séparer le culot de centrifugation et recueillir le surnageant,
- et concentrer, si désiré, le surnageant, pour obtenir une solution constituant ladite fraction de core.

9. Composition selon la revendication 8, **caractérisée par le fait que** ledit tensioactif est choisi parmi les alkylphénols polyoxyéthylénés, les alcools gras polyoxyéthylénés et les alkyl-osides.

10. Composition selon la revendication 5, **caractérisée par le fait que** ladite fraction de core est la protéine M.

11. Composition selon l'une quelconque des revendications précédentes, **caractérisée par le fait qu'**elle se présente sous la forme d'une dose unitaire contenant une quantité efficace dudit additif.

12. Composition selon la revendication précédente, **caractérisée par le fait qu'**elle contient comme additif de 1 à 100 µg de core ou une quantité équivalente de ladite fraction de core.

13. Composition selon la revendication précédente, **caractérisée par le fait qu'**elle contient comme additif de 2 à 100 µg de core, ou une quantité équivalente de fraction de core.

14. Composition selon la revendication précédente, **caractérisée par le fait qu'**elle contient comme additif de 5 à 100 µg de core, ou une quantité équivalente de fraction de core.

15. Composition selon la revendication 11 ou 12, **caractérisée par le fait qu'**elle contient comme additif au moins 3-5 µg de protéine M.

16. Composition selon la revendication précédente, **caractérisée par le fait qu'**elle contient comme additif au moins 7-10 µg de protéine M.

17. Composition selon l'une quelconque des revendications 11 à 15, **caractérisée par le fait que** son constituant vaccin classique contient de 1 à 20 µg d'hémagglutinine de chacune des souches vaccinales qui le composent.

18. Utilisation dans la préparation d'une composition vaccinale contre la grippe, contenant un premier constituant correspondant à un vaccin antigrippal classique à virions entiers, d'un second constituant au mélange avec ledit premier constituant, pour obtenir une composition vaccinale à effet synergique, ledit second constituant étant un additif à effet synergique choisi parmi : des particules de core isolées provenant d'au moins une souche de virus grippal, une fraction isolée de core contenant de la protéine M d'au moins un souche de virus grippal, une protéine M d'au moins une souche de virus grippal, et un fragment à effet synergique de protéine M d'au moins une souche de virus grippal.

19. Utilisation dans la préparation d'une composition vaccinale contre la grippe, contenant un premier constituant correspondant à un vaccin antigrippal fractionné classique, d'un second constituant au mélange avec ledit premier constituant pour obtenir une composition vaccinale à effet synergique, ledit second constituant étant un additif à effet synergique choisi parmi : des particules de core isolées provenant d'au moins une souche de virus grippal, une fraction isolée de core contenant de la protéine M d'au moins un souche de virus grippal, une protéine M d'au moins une souche de virus grippal, et un fragment à effet synergique de protéine M d'au moins une souche de virus grippal.

20. Utilisation selon la revendication précédente, **caractérisée par le fait que** ledit vaccin classique et/ou ledit additif sont tels que définis dans l'une quelconque des revendications 1 à 17.

## Claims

1. Vaccine composition against influenza containing a mixture of a first and of a second constituent, said first constituent being made up of a conventional whole virion influenza vaccine and said second constituent being an additive having a synergistic effect, chosen from isolated particles of core originating from at least one strain of influenza virus, an isolated fraction of core containing M protein of at least one strain of influenza virus, an M protein of at least one strain of influenza virus, and an M protein fragment, having a synergistic effect, of at least one strain of influenza virus.

2. Vaccine composition against influenza containing a mixture of a first and of a second constituent, said first constituent being made up of a conventional split influenza vaccine, and said second constituent being an additive having a synergistic effect, chosen from isolated particles of core originating from at least one strain of influenza virus, an isolated fraction of core containing M protein of at least one strain of influenza virus, an M protein of at least one strain of influenza virus, and an M protein fragment, having a synergistic effect, of at least one strain of influenza virus.

3. Composition according to any one of the preceding claims, **characterized in that** said additive comprises core obtained after removing the envelope proteins by treating an influenza virus with a protease.

4. Composition according to Claim 3, **characterized in that** said protease is bromelain.

5. Composition according to any one of Claims 1 to 4, **characterized in that** said additive comprises an isolated fraction of influenza virus core containing M protein, or comprises an M protein fragment having a synergistic effect.

6. Composition according to Claim 5, **characterized in that** said additive comprises delipidized particles of core.

7. Composition according to any one of Claims 1 to 5, **characterized in that** said additive comprises core obtained by controlled treatment of an influenza virus with at least one surfactant and then separation of said core according to the usual methods.

8. Composition according to Claim 5, **characterized in that** said core fraction can be obtained using a method consisting in
- treating a core suspension with a surfactant, for example a non-ionic surfactant, at a sufficient concentration and at a sufficiently acid pH to promote separation of the M and NP proteins in the following step,
- subjecting the solution thus treated to centrifugation at a rate sufficient for the NP protein and possible residual particles of core to accumulate in the centrifugation pellet while the M protein remains in the supernatant,
- separating the centrifugation pellet and collecting the supernatant,
- and, if desired, concentrating the supernatant, so as to obtain a solution constituting said core fraction.

9. Composition according to Claim 8, **characterized in that** said surfactant is chosen from polyoxyethylenated alkylphenols, polyoxyethylenated fatty alcohols and alkylsaccharides.

10. Composition according to Claim 5, **characterized in that** said core fraction is the M protein.

11. Composition according to any one of the preceding claims, **characterized in that** it is in the form of a unit dose containing an effective amount of said additive.

12. Composition according to the preceding claim, **characterized in that** it contains, as additive, from 1 to 100 µg of core, or an equivalent amount of said core fraction.

13. Composition according to the preceding claim, **characterized in that** it contains, as additive, from 2 to 100 µg of core, or an equivalent amount of core fraction.

14. Composition according to the preceding claim, **characterized in that** it contains, as additive, from 5 to 100 µg of core, or an equivalent amount of core fraction.

15. Composition according to Claim 11 or 12, **characterized in that** it contains, as additive, at least 3-5 µg of M protein.

16. Composition according to the preceding claim, **characterized in that** it contains, as additive, at least 7-10 µg of M protein.

17. Composition according to any one of Claims 11 to 15, **characterized in that** the conventional vaccine constituent thereof contains from 1 to 20 µg of haemagglutinin of each of the vaccine strains which make it up.

18. Use, in the preparation of a vaccine composition against influenza, containing a first constituent corresponding to a conventional whole virion influenza vaccine, of a second constituent as a mixture with said first constituent, in order to obtain a vaccine composition having a synergistic effect, said second constituent being an additive having a synergistic effect, chosen from: isolated particles of core originating from at least one strain of influenza virus, an isolated fraction of core containing M protein of at least one strain of influenza virus, an M protein of at least one strain of influenza virus, and an M protein fragment, having a synergistic effect, of at least one strain of influenza virus.

19. Use, in the preparation of a vaccine composition against influenza, containing a first constituent corresponding to a conventional split influenza vaccine, of a second constituent as a mixture with said first constituent, in order to obtain a vaccine composition having a synergistic effect, said second constituent being an additive, having a synergistic effect, chosen from: isolated particles of core originating from at least one strain of influenza virus, an isolated fraction of core containing M protein of at least one strain of influenza virus, an M protein of at least one strain of influenza virus, and an M protein fragment, having a synergistic effect, of at least one strain of influenza virus.

20. Use according to the preceding claim, **characterized in that** said conventional vaccine and/or said additive are as defined in any one of Claims 1 to 17.

## Patentansprüche

1. Impfzubereitung gegen Grippe, enthaltend ein Gemisch, bestehend aus einem ersten und einem zweiten Bestandteil, wobei der Erste aus einem klassischen antigrippalen Vaccin mit vollständigen Virionen besteht und der zweite Bestandteil ein Additiv mit synergistischer Wirkung darstellt, ausgewählt unter isolierten Kern-Partikeln, die aus mindestens einem Stamm des Grippevirus stammen, einer isolierten Fraktion des Kerns, enthaltend das Protein M mindestens eines Stammes von Grippeviren, einem Protein M mindestens eines Stammes von Grippeviren und einem Fragment mit synergistischer Wirkung des Proteins M mindestens eines Stammes von Grippeviren.

2. Impfzubereitung gegen Grippe, enthaltend ein Gemisch, bestehend aus einem ersten und einem zweiten Bestandteil, wobei der Erste aus einem fraktionierten, klassischen antigrippalen Vaccin besteht und der zweite Bestandteil ein Additiv mit synergistischer Wirkung darstellt, ausgewählt unter isolierten Kern-Partikeln, die aus mindestens einem Stamm des Grippevirus stammen, einer isolierten Fraktion des Kerns, enthaltend das Protein M mindestens eines Stammes von Grippeviren, einem Protein M mindestens eines Stammes von Grippeviren und einem Fragment mit synergistischer Wirkung des Proteins M mindestens eines Stammes von Grippeviren.

3. Zubereitung gemäß einem der vorstehenden Ansprüche, **dadurch gekennzeichnet, dass** das Additiv den Kern umfasst, erhalten nach Elimination von Hüllproteinen durch Behandlung eines Grippevirus mit Hilfe einer Protease.

4. Zubereitung gemäß Anspruch 3, **dadurch gekennzeichnet, dass** die Protease das Bromelain ist.

5. Zubereitung gemäß einem der Ansprüche 1 bis 4, **dadurch gekennzeichnet, dass** das Additiv eine isolierte Fraktion des Kerns des Grippevirus umfasst, enthaltend das Protein M, oder ein Fragment des Proteins M mit synergistischer Wirkung.

6. Zubereitung gemäß Anspruch 5, **dadurch gekennzeichnet, dass** das Additiv entfettete Kern-Partikel umfasst.

7. Zubereitung gemäß einem der Ansprüche 1 bis 5, **dadurch gekennzeichnet, dass** das Additiv den Kern umfasst, erhalten durch schonende Behandlung eines Grippevirus mit Hilfe mindestens eines oberflächenaktiven Mittels, anschließende Abtrennung des Kerns gemäß üblicher Verfahren.

8. Zubereitung gemäß Anspruch 5, **dadurch gekennzeichnet, dass** die Kernfraktion durch ein Verfahren erhalten werden kann, bestehend aus
- der Behandlung einer Suspension des Kerns mit Hilfe eines oberflächenaktiven Mittels, beispielsweise eines nicht ionischen Detergens, bei einer ausreichenden Konzentration und einem ausreichend sauren pH-Wert, um die Abtrennung der Proteine M und NP im folgenden Schritt zu favorisieren,
- Unterwerfen der so behandelten Lösung unter eine Zentrifugation mit ausreichender Geschwindigkeit, damit sich das Protein NP und ggf. rückständige Kern-Partikel im Zentrifugationsniederschlag akkumulieren, wobei das Protein M im Überstand verbleibt,
- Abtrennen des Zentrifugationsniederschlags und Sammeln des Überstandes,
- und Konzentrieren, falls gewünscht, des Überstandes zum Erhalt einer Lösung, welche die Kernfraktion darstellt.

9. Zubereitung gemäß Anspruch 8, **dadurch gekennzeichnet, dass** das oberflächenaktive Mittel ausgewählt ist unter den polyethoxylierten Alkylphenolen, den polyethoxylierten Fettalkoholen und den Alkylosiden.

10. Zubereitung gemäß Anspruch 5, **dadurch gekennzeichnet, dass** die Kernfraktion das Protein M darstellt.

11. Zubereitung gemäß einem der vorstehenden Ansprüche, **dadurch gekennzeichnet, dass** sie in Form einer Einheitsdosis vorliegt, enthaltend eine wirksame Menge des Additivs.

12. Zubereitung gemäß dem vorstehenden Anspruch, **dadurch gekennzeichnet, dass** sie als Additiv 1 bis 100 µg Kern oder einer äquivalenten Menge der Kernfraktion enthält.

13. Zubereitung gemäß dem vorstehenden Anspruch, **dadurch gekennzeichnet, dass** sie als Additiv 2 bis 100 µg Kern oder eine äquivalente Menge der Kernfraktion enthält.

14. Zubereitung gemäß dem vorstehenden Anspruch, **dadurch gekennzeichnet, dass** sie als Additiv 5 bis 100 µg Kern oder eine äquivalente Menge der Kernfraktion enthält.

15. Zubereitung gemäß Anspruch 11 oder 12, **dadurch gekennzeichnet, dass** sie als Additiv mindestens 3-5 µg an Protein M enthält.

16. Zubereitung gemäß dem vorstehenden Anspruch, **dadurch gekennzeichnet, dass** sie als Additiv mindestens 7-10 µg an Protein M enthält.

17. Zubereitung gemäß einem der Ansprüche 11 bis 15, **dadurch gekennzeichnet, dass** ihr Bestandteil als klassisches Vaccin 1 bis 20 µg Hemagglutinin eines der Impfstämme enthält, die diesen zusammensetzen.

18. Verwendung bei der Herstellung einer Impfzubereitung gegen Grippe, enthaltend einen ersten Bestandteil, entsprechend einem klassischen antigrippalen Vaccin aus vollständigen Virionen, und einen zweiten Bestandteil, ein Gemisch mit dem Ersten bildend, um eine Impfzubereitung mit synergistischer Wirkung zu erhalten, wobei es sich bei dem zweiten Bestandteil um ein Additiv mit synergistischer Wirkung handelt, ausgewählt unter: isolierten Kern-Partikeln, hervorgehend aus mindestens einem Stamm des Grippevirus, einer isolierten Fraktion des Kerns, enthaltend das Protein M mindestens eines Stammes von Grippeviren, einem Protein M mindestens eines Stammes von Grippeviren und einem Fragment des Proteins M mit synergistischer Wirkung von mindestens einem Stamm von Grippeviren.

19. Verwendung bei der Herstellung einer Impfzubereitung gegen Grippe, enthaltend einen ersten Bestandteil, entsprechend einem fraktionierten, klassischen antigrippalen Vaccin, und einen zweiten Bestandteil, ein Gemisch mit dem Ersten bildend, um eine Impfzubereitung mit synergistischer Wirkung zu erhalten, wobei es sich bei dem zweiten Bestandteil um ein Additiv mit synergistischer Wirkung handelt, ausgewählt unter: isolierten Kern-Partikeln, hervorgehend aus mindestens einem Stamm des Grippevirus, einer isolierten Fraktion des Kerns, enthaltend das Protein M mindestens eines Stammes von Grippeviren, einem Protein M mindestens eines Stammes von Grippeviren und einem Fragment des Proteins M mit synergistischer Wirkung von mindestens einem Stamm von Grippeviren.

20. Verwendung gemäß dem vorstehenden Anspruch, **dadurch gekennzeichnet, dass** das klassische Vaccin und/oder das Additiv wie in einem der Ansprüche 1 bis 17 definiert sind.
